# DEMANDE DE BREVET EUROPEEN

(11) **EP 0 800 821 A2**
(43) Date de publication de la demande: **15.10.1997**
(21) Numéro de dépôt: 97420056.0
(22) Date de dépôt: 04.04.1997
(51) Int. Cl.: A61K 7/48

(54) **Compositions à base de peroxyde de benzoyle et de l'éther monoéthylique du diéthylène glycol; procédé pour la préparation de telles compositions**

(30) Priorité: 10.04.1996 US 630520
(71) Demandeur: GATTEFOSSE S.A., 69800 Saint Priest (FR)
(72) Inventeur: Simard, Véronique, 69003 Lyon (FR); Garlen, David, Summit, 07 410 New Jersey (US); Klein, Kenneth, Fair Lawn, 07 410 New Jersey (US)
(74) Mandataire: Laurent, Michel

(57) **Abrégé**

Composition à base de peroxyde de benzoyle, caractérisée en ce qu'elle comprend essentiellement du peroxyde de benzoyle et un éther monoéthylique du diéthylène glycol, dans lequel au moins 35 % du peroxyde de benzoyle est dissout.

## Description

### Domaine Technique

L'invention concerne une composition à base de peroxyde de benzoyle ainsi qu'une composition floculante à base de peroxyde de benzoyle.

Elle a également pour objet un procédé pour la préparation de ces compositions, qui permet de réduire le caractère explosif du peroxyde de benzoyle.

L'invention a aussi pour objet une composition aqueuse pour le traitement topique de l'acné ou autre désordre dermatologique, comprenant comme agent actif, une composition à base de peroxyde de benzoyle.

Dans la suite de la description et dans les revendications, par l'expression << composition floculante >>, on désigne une dispersion laiteuse dans laquelle les particules de peroxyde de benzoyle sont dispersées de façon extrêmement fine et n'ont plus de consistance solide.

### Techniques antérieures

Il est bien connu d'utiliser le peroxyde de benzoyle comme élément actif dans les compositions topiques destinées au traitement de l'acné ou autres désordres dermatologiques.

Le peroxyde de benzoyle est un peroxyde organique, de formule :

(C₆H₅CO)₂O₂

Les compositions topiques utilisées pour le traitement de la peau requièrent généralement que le peroxyde de benzoyle soit utilisé sous forme de particules, introduites dans un véhicule approprié. Si le peroxyde de benzoyle n'est pas broyé, le produit est alors granuleux et peut irriter la peau. De plus, l'efficacité clinique, qui est fonction de la surface de l'élément actif en contact avec la peau, la surface de l'élément actif étant elle même fonction de la dimension des particules, sera réduite pour une concentration donnée de peroxyde de benzoyle si l'on fait appel à des particules de grandes tailles.

On sait également que le peroxyde de benzoyle est un agent oxydant potentiellement explosif. C'est la raison pour laquelle il est nécessaire de contrôler les conditions de broyage en effectuant l'opération dans un milieu permettant d'éviter l'augmentation excessive de chaleur. Autrement dit, les procédés de fabrication de ce composé sont difficiles, longs et parfois hasardeux.

Les compositions à base de peroxyde de benzoyle pour le traitement de l'acné sont décrites dans le document US-A-4 606 913. Ce document concerne des émulsions à phase interne élevée et leur utilisation comme milieu de suspension pour des produits cosmétiques ou pharmaceutiques. Les émulsions obtenues sont du type eau dans l'huile, et peuvent être utilisées, par exemple, pour fabriquer des crèmes pour le traitement de l'acné comprenant de l'eau, une huile minérale et du peroxyde de benzoyle. Ce document décrit également différentes substances cosmétiques auxiliaires, qui peuvent être employées lorsque les émulsions à phase interne élevée sont utilisées, en tant que véhicule, dans des compositions cosmétiques. Ces substances cosmétiques auxiliaires comprennent des solvants tels que l'éther monoéthylique d'éthylène glycol, l'éther monoéthylique de diéthyléne glycol, l'éther monobutylique de diéthylène glycol et d'autres encore. Ces préparations sont fabriquées en ajoutant la phase aqueuse à la phase huileuse. Les éléments constituant une partie de la phase aqueuse sont ajoutés dans la phase aqueuse avant que les deux phases soient mélangées.

Dans le document US-A-4 671 956, on a décrit des compositions anti-acnéiques combinées avec des filtres solaires. Ces compositions pour le traitement local de l'acné, contiennent de 0,5 à 20 % en poids de peroxyde de benzoyle en association avec au moins un filtre solaire. Ici, le peroxyde de benzoyle est utilisé sous forme de poudre finement divisée à l'état sec ou humide, de préférence à l'état humide. Ces compositions anti-acnéiques se présentent sous forme d'émulsions et sont préparées en ajoutant le peroxyde de benzoyle dans la phase aqueuse tandis que le filtre solaire est ajouté soit dans la phase aqueuse soit dans la phase huileuse en fonction de son affinité pour l'une de ces phases. Dans ces compositions, le propylène glycol est utilisé en tant qu'agent humectant ou solvant, de même que des alcools aliphatiques plus courts peuvent être incorporés dans des préparations de type gel.

Dans le document US-A-5 019 567, on a suggéré de combiner le peroxyde de benzoyle avec des salicylates lipophiliques d'ammonium quaternaire pour la préparation de compositions destinées au traitement des dermatoses, de l'acné, des verrues, de la dékératinisation de la peau, des ulcères cutanés et autres désordres cutanés. Ce document exemplifie des compositions sous forme de gels comprenant un monoéthyl éther de diéthylène glycol comme constituant parmi d'autres, mais ne décrit pas la méthode pour préparer de tels gels.

Dans le document US-A-5 460 620, on a décrit un dispositif destiné à être appliqué sur la peau, permettant de dispenser un principe actif. Dans les exemples d'application, le peroxyde de benzoyle est utilisé en tant que principe actif pour le traitement de l'acnée, mais il n'y a aucune information relative à sa forme d'utilisation. Par ailleurs, l'ether monoéthylique de diéthylène glycol est utilisé ici, en tant que promoteur d'absorption pour améliorer la diffusion du principe actif.

On a découvert et c'est l'objet de l'invention, un procédé perfectionné de préparation d'une composition à base de peroxyde de benzoyle consistant en la fabrication d'un mélange, qui peut être ensuite ajouté à un milieu aqueux, afin d'obtenir une nouvelle composition floculante, lui conférant un toucher lisse ou impalpable, lorsqu'elle est étendue sur la peau.

Cette méthode est plus facile à mettre en oeuvre, plus rapide et plus sûre que les techniques conventionnelles dans lesquelles on broyait le peroxyde de benzoyle pour obtenir des fines particules, d'autant que les particules de peroxyde de benzoyle dans ladite composition floculante sont sensiblement plus petites que celles obtenues par les techniques conventionnelles de broyage.

### Description sommaire de l'invention

L'invention concerne de nouvelles compositions à base de peroxyde de benzoyle qui se caractérisent en ce qu'elles comprennent essentiellement du peroxyde de benzoyle et un éther monoéthylique du diéthylène glycol, dans lequel au moins 35 % du peroxyde de benzoyle est dissout.

On a découvert en effet, que la dissolution du peroxyde de benzoyle dans un solvant spécifique, à savoir un éther monoéthylique du diéthylène glycol permet non seulement de réduire de manière importante le caractère explosif du peroxyde de benzoyle, mais également d'obtenir des particules de peroxyde de benzoyle extrêmement fines.

En tant qu'éther monoéthylique du diéthylène glycol, on utilise le produit du Demandeur, commercialisé sous la marque TRANSCUTOL®

Selon une première caractéristique de l'invention, au moins 50 % du peroxyde de benzoyle est dissout dans un éther monoéthylique de diéthylène glycol, de préférence aux environs de 100%.

Par ailleurs, le rapport en poids entre le peroxyde de benzoyle et l'éther monoéthylique de diéthylène glycol varie entre 1:1 et 1:18, de préférence entre 1:2 et 1:5.

Afin de réduire le caractère explosif du peroxyde de benzoyle, on dissout partiellement ou complètement le peroxyde de benzoyle dans un éther monoéthylique du diéthylène glycol, sous agitation, et/ou chauffage à une température comprise entre 20 et 50°C, avantageusement 45°C.

A une température inférieure à 20°C, on n'observe pas de dissolution. A une température supérieure à 50°C, les risques d'explosion deviennent importants.

L'invention concerne également un composition floculante à base de peroxyde de benzoyle, caractérisée en ce qu'elle comprend essentiellement du peroxyde de benzoyle et un éther monoéthylique du diéthylène glycol, dans lequel au moins 35 % du peroxyde de benzoyle est dissout, ainsi que de l'eau ou un milieu aqueux.

La dissolution du peroxyde de benzoyle dans un éther monoéthylique du diéthylène glycol permet d'obtenir des particules extrêmement fine ce qui augmente sa biodisponibilité au niveau de la peau. De plus la composition obtenue présente un toucher lisse lorsqu'elle est appliquée sur la peau.

De même que précédemment, afin de réduire le caractère explosif du peroxyde de benzoyle, on dissout partiellement ou complètement le peroxyde de benzoyle dans un éther monoéthylique du diéthylène glycol, sous agitation, et/ou chauffage à une température comprise entre 20 et 50°C, puis on mélange la solution obtenue avec de l'eau ou un milieu aqueux.

L'invention concerne également une composition aqueuse pour le traitement topique de l'acné ou autre désordre dermatologique comprenant comme élément actif une composition à base de peroxyde de benzoyle, caractérisée en ce qu'elle comprend essentiellement du peroxyde de benzoyle et un ether monoéthylique de diéthylène glycol ,dans lequel au moins 35 % du peroxyde de benzoyle est dissout.

En d'autres termes, les compositions selon l'invention sont utilisées pour le traitement de l'acné ou pour d'autres désordres dermatologiques connus pour être sensibles au peroxyde de benzoyle. La fine dimension des particules de peroxyde de benzoyle des compositions de l'invention, améliorent l'efficacité du peroxyde de benzoyle, car plus les particules sont petites, plus on augmente la surface de contact des éléments actifs sur la peau et donc la biodisponibilité du produit.

Avantageusement, cette composition pour le traitement topique de l'acné ou autres désordres dermatologiques, se présente sous forme de lotion, de pommade, gel ou de crème.

### Manière de réaliser l'invention

Dans la description, tous les pourcentages et rapports sont exprimés en poids.

Le terme « mélange » tel qu'il est utilisé dans la présente description et dans les revendications, englobe non seulement des opérations classiques de mélange, mais également les opérations équivalentes tels que combinaisons, dissolutions ou similaires.

Dans les exemples de réalisation, comme éther monoéthylique de diéthylène glycol, on utilise un composé commercialisé par le Demandeur sous la marque déposée TRANSCUTOL® de formule chimique :

C₂H₅-O-CH₂-CH₂-O-CH₂-CH₂-OH

purifié par rectification, de poids moléculaire 134,17 se présentant sous la forme d'un liquide limpide ayant une faible odeur éthérée, conditionnée sous azote, bien connu comme promoteur d'absorption.

Les compositions à base de peroxyde de benzoyle selon l'invention, sont préparées en mélangeant du peroxyde de benzoyle et du TRANSCUTOL®, et en dissolvant partiellement, de préférence complètement le peroxyde de benzoyle dans le TRANSCUTOL®.

Comme déjà dit, le rapport en poids entre le peroxyde de benzoyle et l'éther monoéthylique de diéthylène glycol varie entre 1:1 et 1:18, de préférence entre 1:2 et 1:5, avantageusement entre 1:2 et 1:4.

Bien qu'il soit préférable de dissoudre la totalité du peroxyde de benzoyle, une dissolution incomplète conduit à de bons résultats. On obtient ainsi une composition qui, lorsqu'elle est incorporée dans une composition topique appropriée, présente une aspect lisse sur la peau.

Toutefois, en pratique, il faut qu'au moins 35 % de peroxyde de benzoyle soit dissout dans le TRANSCUTOL®, de préférence au moins 50 %, et avantageusement aux environs de 100 %.

On réduit le temps nécessaire à la dissolution du peroxyde de benzoyle dans le TRANSCUTOL® en augmentant l'agitation et en augmentant la température. Avantageusement on opère au voisinage de 45°C,.sans toutefois excéder une température de 50°C, et ainsi éviter une décomposition violente du peroxyde de benzoyle.

Lorsque le mélange est obtenu, on peut l'utiliser directement pour la préparation de compositions pour application topique sur la peau.

Ces compositions sont préparées en mélangeant le mélange ci-avant avec de l'eau, afin d'obtenir une composition flocculante, avant d'y ajouter les autres constituants.

En variante, le mélange de peroxyde de benzoyle et de TRANSCUTOL® peut être mélangé avec un milieu aqueux contenant de l'eau et d'autres constituants, et ce mélange devient alors la phase aqueuse d'une émulsion eau dans huile ou huile dans eau.

### Exemple 1

On mélange cinq parties de peroxyde de benzoyle avec dix parties d'un éther monoéthylique de diéthylène glycol, commercialisé par le Demandeur sous la marque TRANSCUTOL®, pour former une dispersion. La quasi-totalité du peroxyde de benzoyle est alors dissout.

On ajoute cette dispersion à 85 parties d'eau à température ambiante. On obtient une dispersion laiteuse, c'est-à-dire une composition floculante.

Lorsque la dispersion laiteuse est étendue sur la peau, aucune impureté n'est observée, et on ne ressent pas la présence de poudre. A l'examen microscopique, on observe des particules de peroxyde de benzoyle extrêmement petites.

### Exemple 2

A titre comparatif, on mélange 5 parties de peroxyde de benzoyle avec 95 parties d'eau. Lorsque ce mélange est étendu sur la peau, on observe des particules rugueuses et grattantes.

A l'examen microscopique, on observe des particules de peroxyde de benzoyle de tailles relativement importantes, en tous cas de plus de dix fois celles observées à l'exemple 1.

### Exemple 3

On étudie la cinétique de dissolution du peroxyde de benzoyle dans l'éther monoéthylique de diéthylène glycol.

5 grammes de peroxyde de benzoyle sont mélangés avec 95 grammes d'éther monoéthylique de diéthylène glycol au moyen d'un mélangeur à vis tournant, à 500 tours/minute, à 20°C. Après sept minutes, on observe que 90 % du peroxyde de benzoyle est dissout et après dix minutes, environ 100 %.

## Revendications

1. Composition à base de peroxyde de benzoyle, caractérisée en ce qu'elle comprend essentiellement du peroxyde de benzoyle et un éther monoéthylique du diéthylène glycol, dans lequel au moins 35 % du peroxyde de benzoyle est dissout.

2. Composition selon la revendication 1, caractérisée en qu'au moins 50 % du peroxyde de benzoyle est dissout dans un éther monoéthylique de diéthylène glycol, de préférence aux environs de 100%.

3. Composition selon l'une des revendications 1 et 2, caractérisée en ce que le rapport en poids entre le peroxyde de benzoyle et l'éther monoéthylique de diéthylène glycol varie entre 1:1 et 1:18, de préférence entre 1:2 et 1:5.

4. Composition floculante à base de peroxyde de benzoyle, caractérisée en ce qu'elle comprend essentiellement du peroxyde de benzoyle et un éther monoéthylique du diéthylène glycol, dans lequel au moins 35 % du peroxyde de benzoyle est dissout, ainsi que de l'eau ou un milieu aqueux.

5. Procédé pour la préparation d'une composition à base de peroxyde de benzoyle, caractérisé en ce qu'on dissout partiellement ou complètement le peroxyde de benzoyle dans un éther monoéthylique du diéthylène glycol, sous agitation, et/ou chauffage à une température comprise entre 20 et 50°C.

6. Procédé pour la préparation d'une composition floculante à base de peroxyde de benzoyle, caractérisée en ce qu'on dissout partiellement ou complètement le peroxyde de benzoyle dans un éther monoéthylique du diéthylène glycol, sous agitation, et/ou chauffage à une température comprise entre 20 et 50°C, puis on mélange la solution obtenue avec de l'eau ou un milieu aqueux.

7. Composition aqueuse pour le traitement topique de l'acné ou autre désordre dermatologique comprenant comme élément actif une composition à base de peroxyde de benzoyle, caractérisée en ce qu'elle comprend essentiellement du peroxyde de benzoyle et un ether monoéthylique de diéthylène glycol, dans lequel au moins 35 % du peroxyde de benzoyle est dissout.

8. Composition aqueuse selon la revendication 7, caractérisée en ce qu'elle se présente sous forme de lotion, de pommade, de gel ou de crème.
